# EUROPEAN PATENT SPECIFICATION

(11) **EP 2 566 473 B1**
(45) Date of publication and mention of the grant of the patent: **06.01.2016**
(21) Application number: 11720419.8
(22) Date of filing: 05.05.2011
(51) Int. Cl.: A61K 31/145, A61P 37/08

(54) **DOSAGE REGIMEN OF DIARYL SULFIDE DERIVATIVES**
Dosierungsplan von Diarylsulfidderivaten
Posologie de dérivés de sulfure de diaryle

(30) Priority: 06.05.2010 EP 10162082
(43) Date of publication of application: 13.03.2013
(73) Proprietor: Novartis AG, 4056 Basel (CH)
(72) Inventor: GERGELY, Peter, CH-4002 Basel (CH); LEGANGNEUX, Eric, CH-4002 Basel (CH); WALLSTROEM, Erik, CH-4002 Basel (CH)
(74) Representative: von Sprecher, Georg
(86) International application number: PCT/EP2011/057210
(87) International publication number: WO 2011/138398

(56) References cited:
- EP-A1- 1 431 284
- EP-A1- 1 548 003
- EP-A1- 1 772 145
- WO-A1-2010/075239
- WO-A2-2007/028821
- SHIMIZU HISASHI ET AL: "KRP-203, a novel synthetic immunosuppressant, prolongs graft survival and attenuates chronic rejection in rat skin and heart allografts", CIRCULATION, vol. 111, no. 2, 18 January 2005 (2005-01-18), pages 222-229, XP002587366, ISSN: 0009-7322
- OPPENHEIMER FEDERICO ET AL: "Impact of long-term therapy with FTY720 or mycophenolate mofetil on cardiac conduction and rhythm in stable adult renal transplant patients", TRANSPLANTATION (HAGERSTOWN), vol. 83, no. 5, March 2007 (2007-03), pages 645-648, XP002587367, ISSN: 0041-1337

## Description

### Field of the Invention

The present invention relates to a dosage regimen for certain diaryl sulfide derivatives or agonists useful as immunosuppressive or immunomodulating agents. More specifically, the present invention relates to a dosage regimen using these diaryl sulfide derivatives for the treatment of patients suffering from autoimmune diseases or disorders.

### Background of the Invention

The diaryl sulfide derivatives for use in the invention are S1 P receptor modulators or agonists which signal as agonists at one or more sphingosine-1 phosphate receptors, for example, S1 P1 to S1 P8. The binding of an agonist to a S1 P receptor may, for example, result in the dissociation of intracellular heterotrimeric G-proteins into Gα-GTP and Gβγ-GTP, and/or the increased phosphorylation of the agonist-occupied receptor, and/or the activation of downstream signaling pathways/kinases.

S1 P receptor modulators or agonists are useful therapeutic compounds for the treatment of various conditions in mammals, especially in human beings. For example, the efficacy of S1 P receptor modulators or agonists in the prevention of transplant rejection has been demonstrated in rat (skin, heart, liver, small bowel), dog (kidney), and monkey (kidney) models. In addition, due to their immune-modulating potency, S1 P receptor modulators or agonists are also useful for the treatment of inflammatory and autoimmune diseases. In particular, the efficacy of the S1 P receptor agonist FTY720 in the treatment of multiple sclerosis has been demonstrated in humans (as described in, for example, "FTY720 therapy exerts differential effects on T cell subsets in multiple sclerosis". Mehling M, Brinkmann V, Antel J, Bar-Or A, Goebels N, Vedrine C, Kristofic C, Kuhle J, Lindberg RL, Kappos L. Neurology. 2008 Oct 14;71(16):1261-7; and "Oral fingolimod (FTY720) for relapsing multiple sclerosis". Kappos L, Antel J, Comi G, Montalban X, O'Connor P, Polman CH, Haas T, Korn AA, Karlsson G, Radue EW; FTY720 D2201 Study Group. N Engl J Med. 2006 Sep 14;355(11):1124-40.).

S1P receptor modulators or agonists may produce a negative chronotropic effect e.g. at therapeutic doses, i.e. they may reduce the cardiac rhythm, as described e.g. in "FTY720: Placebo-Controlled Study of the Effect on Cardiac Rate and Rhythm in Healthy Subjects", Robert Schmouder, Denise Serra, Yibin Wang, John M. Kovarik, John DiMarco, Thomas L. Hunt and Marie-Claude Bastien. J. Clin. Pharmacol. 2006; 46; 895. Administration of 1.25 mg of FTY720 may induce a decrease in heart rate of approximately 8 beats/min (BPM).

As a consequence of this side effect, some S1 P modulator or agonist therapy may have to be initiated under close medical supervision in order to check that the cardiac rhythm is maintained at an acceptable level. This may involve the hospitalisation of patients, which makes the treatment more expensive and complicated.

The diaryl sulfide derivatives of the invention are generally well tolerated and give rise to a comparatively mild negative chronotropic effect. However, even a mild negative chronotropic effect can give rise to some patient inconvenience, for example in patients having a low resting heart rate. Such inconvenience can affect patient complience e.g. by acting as a disincentive to resume treatment following a treatment holiday.

In addition, a reduction in the negative chronotropic effect may give enable a broader dosage range to be used, since the observed negative chronotropic effect often increases with increased dosage.

Therefore, there is a need to reduce the negative chronotropic side effect that may be generated by the administration of the above diaryl sulfide derivatives, while maintaining the ability to administer an adequate dosage in order to treat or prevent the diseases for which the compound is administered. There is furthermore a need to enhance patient compliance.

### Brief Disclosure of the Invention

The compounds of the invention are covered by formula I: 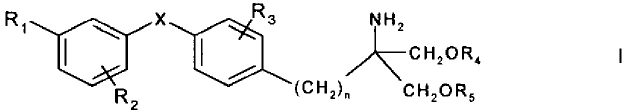
wherein X is O, S, SO or SO₂;
R₁ is halogen, trihalomethyl, -OH, C₁₋₇alkyl, C₁₋₄alkoxy, trifluoromethoxy, phenoxy, cyclohexylmethyloxy, pyridylmethoxy, cinnamyloxy, naphthylmethoxy, phenoxymethyl,-CH₂-OH, -CH₂-CH₂-OH, C₁₋₄alkylthio, C₁₋₄alkylsulfinyl, C₁₋₄alkylsulfonyl, benzylthio, acetyl, nitro or cyano, or phenyl, phenylC₁₋₄alkyl or phenyl-C₁₋₄alkoxy each phenyl group thereof being optionally substituted by halogen, CF₃, C₁₋₄alkyl or C₁₋₄alkoxy;
R₂ is H, halogen, trihalomethyl, C₁₋₄alkoxy, C₁₋₇alkyl, phenethyl or benzyloxy;
R₃ H, halogen, CF₃, OH, C₁₋₇alkyl, C₁₋₄alkoxy, benzyloxy, phenyl or C₁₋₄alkoxymethyl; each of R₄ and R₅, independently is H or a residue of formula (a) 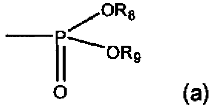
wherein each of R₈ and R₉, independently, is H or C₁₋₄alkyl optionally substituted by halogen;
and n is an integer from 1 to 4;
or a pharmaceutically acceptable salt, hydrate, solvate, isomer or prodrug thereof;
or a compound of formula II 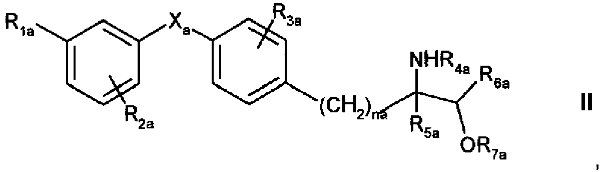 wherein
   - R₁ₐ: is halogen, trihalomethyl, C₁₋₄alkyl, C₁₋₄alkoxy, C₁₋₄alkylthio, C₁₋₄alkylsulifinyl, C₁₋₄alkyl-sulfonyl, aralkyl, optionally substituted phenoxy or aralkyloxy;
   - R₂ₐ: is H, halogen, trihalomethyl, C₁₋₄alkyl, C₁₋₄alkoxy, aralkyl or aralkyloxy;
   - R₃ₐ: is H, halogen, CF₃, C₁₋₄alkyl, C₁₋₄alkoxy, C₁₋₄alkylthio or benzyloxy;
   - R₄ₐ: is H, C₁₋₄alkyl, phenyl, optionally substituted benzyl or benzoyl, or lower aliphatic C₁₋₅acyl;
   - R₅ₐ: is H, monohalomethyl, C₁₋₄alkyl, C₁₋₄alkoxy-methyl, C₁₋₄alkyl-thiomethyl, hydroxyethyl, hydroxypropyl, phenyl, aralkyl, C₂₋₄alkenyl or -alkynyl;
   - R₆ₐ: is H or C₁₋₄alkyl;
   - R₇ₐ: is H, C₁₋₄alkyl or a residue of formula (a) as defined above,
   - Xₐ: is O, S, SO or SO₂; and
   - nₐ: is an integer of 1 to 4;
or a pharmaceutically acceptable salt, hydrate, solvate, isomer or prodrug thereof, and are for use in a method of treatment or prophylaxis, optionally of an autoimmune condition, wherein the compound of the invention is administered at a dosage lower than the standard daily dosage of said compound during the initial period of treatment and then is increased, optionally stepwise, up to the standard daily dosage.

In accordance with the disclosure , there is provided a method for assessing the need or suitability of a patient for a treatment regimen as described in any of the specified aspects or embodiments of the invention comprising the steps of:
(i) determining whether the patient to be treated with the compound of formula I or II, or a pharmaceutically acceptable salt, hydrate, solvate, isomer or prodrug thereof, as defined in the first aspect, is in a category for which the use of a treatment regimen as described above may be beneficial; and
(ii) if the patient falls within this category, treating the patient using a treatment regimen as described above.

The patient may be in the above category if he or she suffers from or is susceptible to heart failure, arrhythmias, high grade atrio-ventricular blocks or sick sinus syndrome or has a history of syncopal episodes; or is undergoing beta blocker or anti-arrhythmic treatment, e.g. is under treatment with anti-arrhythmic drugs; or has undergone an interruption or treatment holiday in the maintenance dosage regime e.g. a holiday of greater than 4 days, greater than 6, 8, 10, 12 or 14 days.

In a further aspect of the invention, there is provided a kit containing units, optionally daily units, of medication of a compound of the invention as defined in the first aspect, of varying daily dosage, whereby said doses are lower than the standard daily dosage. In a further aspect of the invention, there is provided a kit comprising units of medication, optionally daily units, of a compound of the invention as defined in the first aspect for administration according to the dosage regimen defined in any of the aspects or embodiments of the invention, whereby one or more low-dose units of a dose strength below the standard daily dose of said compound are provided for the initial period of treatment.

Further aspects and embodiments are provided in the detailed disclosure of the invention.

### Detailed Disclosure of the Invention

Surprisingly it has been found that by administering the compounds of the invention according to a specific dosage regimen, it is possible to reduce side effects which may be associated with the administration of such compounds. For example, administering the compounds according to the specific dosage regimen of the present invention may significantly reduce, or even completely eliminate, the negative chronotropic side effect. In particular, it may avoid an abrupt drop in the heart rate.

Administering these compounds according to the specific dosage regimen of the present invention may also significantly reduce or even completely eliminate the risk that the patient taking the compounds suffers from heart effects (e.g. Atrio-ventricular (AV) blocks) or heart pauses e.g. heart pauses greater than 2 seconds. Reduction in heart effects or pauses includes reduction in severity, frequency and/or duration. Where the heart effect is an AV block, the term "AV block" include the categories AV-block I, AV-block II Mobitz I/Wenckebach, AV-block II Mobitz II and AV-block II.

Additional benefits of the invention may include further reduction of the risk of other possible adverse effects relating e.g. to variation in blood pressure, renal effects (e.g. as measured by asymptotic elevation of liver enzymes) or pulmonary effects.

Furthermore the specific dosage regimen of the present invention may permit the administration of the compounds of the invention to categories of patients for which the risk/benefit ratio may otherwise be less favourable. Such patients could for example include patients suffering from or susceptible to heart problems e.g. heart failure or arrhythmias, patients suffering from or susceptible to high grade atrio-ventricular blocks or sick sinus syndrome, patients with a history of syncopal episodes, or patients undergoing beta blocker or anti-arrhythmic treatment, such as patients under treatment with anti-arrhythmic drugs; or patients that have undergone an interruption or treatment holiday in the maintenance dosage regime e.g. a holiday of greater than 4 days, greater than 6, 8, 10, 12 or 14 days.

The dosage regimen of the present invention is a regimen for the initiation of therapy, which enables the standard daily therapeutic dosage of the compounds to be achieved with minimal negative chronotropic effects and/or the AV block effects possibly associated with S 1 P receptor modulator therapy.

### Compound of formula I or II

With regard to the compounds of formulae (I) and (II), the term "halogen" encompasses fluorine, chlorine, bromine and iodine. The term "trihalomethyl" encompasses trifluoromethyl and trichloromethyl. "C₁₋₇ alkyl" encompasses straight-chained or branched alkyl, e.g. methyl, ethyl, propyl, isopropyl, butyl, *t*-butyl, pentyl, hexyl or heptyl. The phrase "optionally substituted phenoxy" encompasses unsubstituted phenoxy groups and those that have, at any position of its benzene ring, a halogen atom, such as fluorine, chlorine, bromine and iodine, trifluoromethyl, C₁₋₄alkyl or C₁₋₄alkoxy. The term "aralkyl" as in "aralkyl group" or "aralkyloxy group" encompasses benzyl, diphenylmethyl, phenethyl and phenylpropyl. Any C₁₋₄ alkyl moiety e.g. as present in "C₁₋₄alkoxy", "C₁₋₄alkylthio", "C₁₋₄alkylsulfinyl" or "C₁₋₄alkylsulfonyl" encompasses straight-chained or branched C₁₋₄alkyl, e.g. methyl, ethyl, propyl, isopropyl or butyl. The phrase "optionally substituted aralkyl group" encompasses unsubstituted aralkyl groups and those that have, at any position of its benzene ring, a halogen atom, such as fluorine, chlorine, bromine and iodine, trifluoromethyl, lower alkyl having 1-4 carbon atoms, or lower alkoxy having 1-4 carbon atoms.

More specifically defined compounds within the compounds of formula I are compounds of formula **Ia** 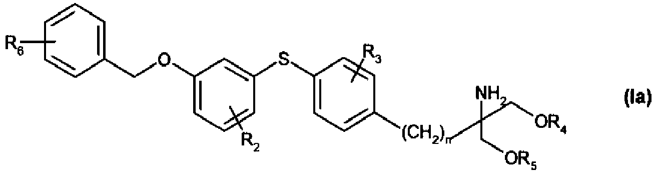 wherein
R₂, R₃, R₄, R₅ and n are as defined above; and
R₆ is hydrogen, halogen, C₁₋₇alkyl, C₁₋₄alkoxy or trifluoromethyl.

The actual compounds of the invention are 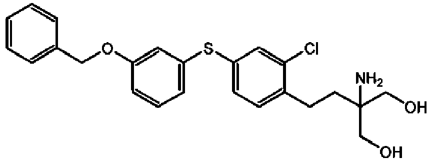 2-amino-2-[4-(3-benzyloxyphenylthio)-2-chlorophenyl]ethyl-propane-1,3-diol and its corresponding phosphate derivative, phosphoric acid mono-2-amino-2-[4-(3-benzyloxyphenylthio)-2-chlorophenyl]ethyl-propyl] ester.

The phosphoric acid mono-2-amino-2-[4-(3-benzyloxyphenylthio)-2-chlorophenyl]ethyl-propyl] ester can be prepared enantiomerically pure by the procedures described in WO 2005/021503 to give:

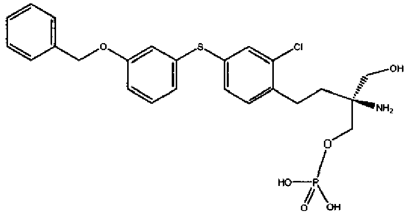

Phosphoric acid mono-{(S)-2-amino-4-[4-(3-benzyloxy-phenylsulfanyl)-2-chloro-phenyl]-2-hydroxymethyl-butyl}ester or

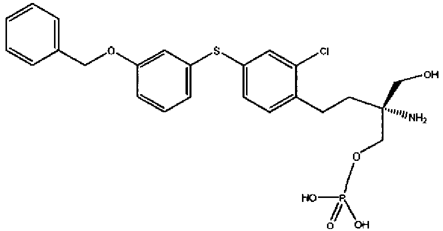

Phosphoric acid mono-{(R)-2-amino-4-[4-(3-benzyloxy-phenylsulfanyl)-2-chloro-phenyl]-2-hydroxymethyl-butyl}ester

More specifically defined compounds of formula II are compounds of formula (IIa) 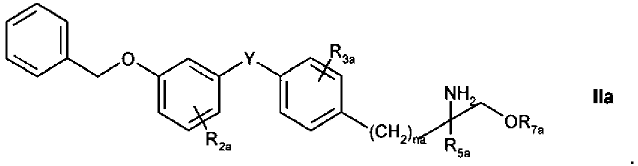 wherein
Y is O or S; and
R₂ₐ, R₃ₐ, R₅ₐ, R₇ₐ and nₐ are as defined above.

Specific compounds of formula (IIa) are those wherein R₃ is chlorine, e.g., 2-amino-4-[4-(3-benzyloxyphenylthio)-2-chlorophenyl]-2-methylbutane-1-ol; the corresponding phosphoric acid mono-2-amino-4-[4-(3-benzyloxyphenylthio)-2-chlorophenyl]-2-methylbutyl] ester; 2-amino-4-[4-(3-benzyloxyphenylthio)-2-chlorophenyl]-2-ethylbutane-1-ol; and the corresponding phosphoric acid mono-2-amino-4-[4-(3-benzyloxyphenylthio)-2-chlorophenyl]-2-ethylbutyl] ester. Compounds of formulae I and II are known and are disclosed e.g. in WO03/029205, WO 03/029184 and WO04/026817, respectively, the phosphorylated derivatives being disclosed e.g. in WO04/074297

Compounds of formulae I and II may be prepared as disclosed in above cited references.

Phosphorylated derivatives of compounds of formula (I), e.g., phosphoric acid mono-2-amino-2-[4-(3-benzyloxyphenylthio)-2-chlorophenyl]ethyl-propyl] ester, can be prepared utilizing the procedures for synthesizing phosphorylated compounds described e.g., in WO 2005/021503 (see, e.g., pages 11 and 12). Optically active compounds of structural formula (I) and phosphorylated derivatives thereof, in particular of formula (Ia) can be prepared in high purity utilizing the procedure described, e.g., in Hinterding et al., Synthesis, Vol. 11, pp.1667-1670 (2003). As an example, an optically active compound of structural formula (Ia), phosphoric acid mono-2-amino-2-[4-(3-benzyloxyphenylthio)-2-chlorophenyl]ethyl-propyl] ester, can be prepared as described in the scheme below utilizing the procedures of Hinterding et al. (2003) *supra.* 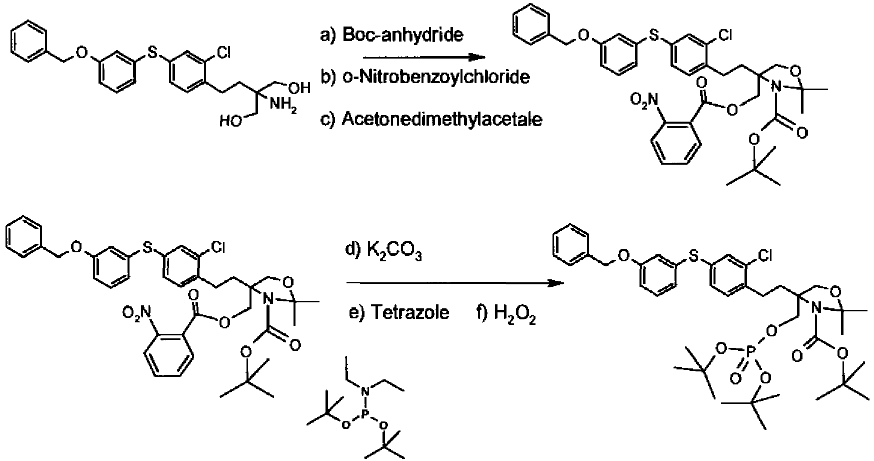
a) 1 equivalent of compound 1 and 1.2 equivalents Boc-anhydride in dioxane/acetonitrile or DMF/water (depends on solubility) + 1.2 equivalents NaOH 1 M in water (RT, overnight).
b) 1 equivalent of step a), 1.5 equivalents 2-nitrobenzoylchloride and 1.6 equivalents pyridine in CH₂Cl₂ (RT, overnight).
c) 1 equivalent of step b), 3 equivalents acetonedimethylacetale and 0.1 equivalents p-TsOH•H₂O in toluene (95°C, 3 hours).
d) 1 equivalent of step c) and 0.075 equivalents K₂CO₃ (powder) in MeOH/THF (1/1) (RT, 4 hours).
e) 1 equivalent of step a), 6 equivalents tetrazole (recrystallized from toluene or 0.45 M in CH₃CN) and 2 equivalents di-*t*-butyldiethylphosphoramidite in dry THF (RT, 3 hours).
f) 5 equivalents H₂O₂ (30%) directly into the reaction mixture of step e) (0°C, 1 hour).

Isolation: the reaction mixture is quenched with sodium thiosulfate (saturated in water) and extracted with ethyl acetate (3x).

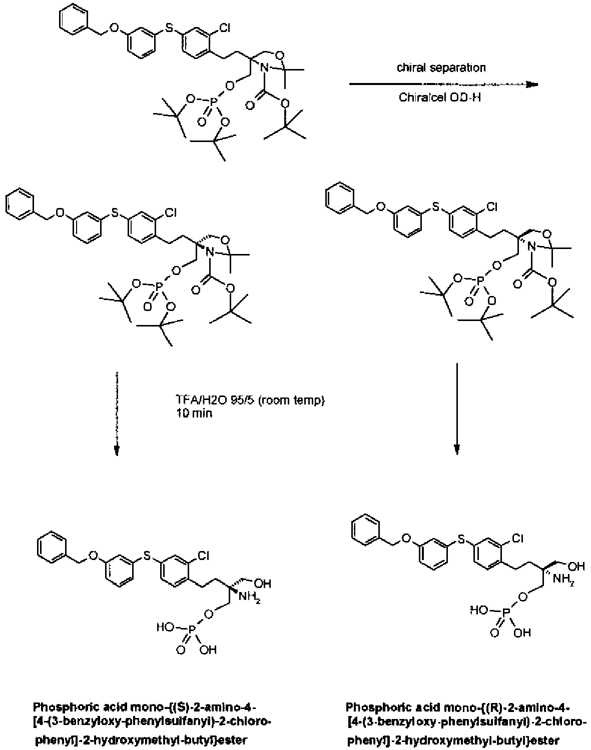

The compounds of formulae II and IIa, e.g., 2-amino-4-[4-(3-benzyloxyphenylthio)-2-chlorophenyl]-2-methylbutane-1-ol and 2-amino-4-[4-(3-benzyloxyphenylthio)-2-chlorophenyl]-2-ethylbutane-1-ol can be prepared as described e.g., in EP 1 548 003 A1. Preparation of such compounds of formulae II and IIa in high optical purity, can be prepared by the procedures described e.g., in Hinterding et al. (2003), *supra;* and Hinterding et al., Tetra Lett, Vol. 43, No. 45, pp. 8095-8097 (2002). Optically active phosphate derivatives of compounds of structural formulae II and IIa, e.g., phosphoric acid mono-2-amino-4-[4-(3-benzyloxyphenylthio)-2-chlorophenyl]-2-methylbutyl] ester and phosphoric acid mono-2-amino-4-[4-(3-benzyloxyphenylthio)-2-chlorophenyl]-2-ethylbutyl] ester can be prepared in high purity as described in Hinterding et al. (2003), *supra.*

The compounds of formulae I and II may exist in free form or salt form, or as a prodrug, solvate or hydrate.

Examples of pharmaceutically acceptable salts of the compounds of the formulae I and II include salts with inorganic acids, such as hydrochloride and hydrobromide salts and salts with organic acids, such as acetate, trifluoroacetate, citrate, tartrate and methanesulfonate salts.

When the compounds of formula I and II have one or more asymmetric centers in the molecule various optical isomers are obtained. The present invention embraces enantiomers, racemates, diastereoisomers and mixtures thereof. Moreover, when compounds of formula I and II include geometric isomers, the present invention embraces cis-compounds, trans-compounds and mixtures thereof.

The disclosure provides forms of the compound that have a hydroxyl or amine group present in a protected form; these function as prodrugs. Prodrugs are compounds that are converted into an active drug form after administration, through one or more chemical or biochemical transformations. Forms of the compounds of the present invention that are readily converted into the claimed compound under physiological conditions are prodrugs of the claimed compounds and are within the scope of the present disclosure.

Examples of prodrugs include forms where a hydroxyl group is acylated to form a relatively labile ester such as an acetate ester, and forms where an amine group is acylated with the carboxylate group of glycine or an L-amino acid such as serine, forming an amide bond that is particularly susceptible to hydrolysis by common metabolic enzymes.

### Dosage Regimens

As previously stated, the present invention provides a novel dosage regimen which is adapted to ameliorate or minimize the negative chronotropic effects and/or the heart effects possibly associated with therapy using the compounds of the invention.

Heart effects include AV blocks, which include first degree AV blocks (e.g. PR intervals greater then 0.2 seconds) and second degree AV blocks e.g. first degree AV blocks. Heart effects include heart pauses e.g. heart pauses greater than 2 seconds.

In the aspects of the invention, for example during the entire initial period of treatment or on the day that the initial therapeutic dose is administered, the medication is administered in a dosage regimen such that the daily decrease in heart rate (e.g. average or minimum daily heart rate) is acceptable or clinically not significant, or that the sinus rhythm of the patient is normal. For example, the daily decrease in heart rate (e.g. average or minimum daily heart rate) may be less than about 4bpm, e.g. less than about 3 bpm or less than about 2bpm.

The term "normal sinus rhythm" refers to the sinus rhythm of the patient when not undergoing treatment. The evaluation of normal sinus rhythm is within the ability of a physician. A normal sinus rhythm will generally give rise to a heart rate in the range from 60-100 bpm.

According to the invention, the "initial period of treatment" refers to the period during which the compound of the invention is administered at a dosage lower than the standard daily dosage. Preferably the "initial period of treatment" starts with the first administration of the compound.

As herein above defined, standard daily dosage (also called standard daily dose) refers to the daily maintenance dose of the drug which is given to the patients for treating or preventing the disease to be treated or prevented. Preferably, the standard daily dosage corresponds to the therapeutic dosage.

The therapeutically effective dosage (also called therapeutic dose) refers to the dosage of the compound of the invention which is necessary to effectively treat the intended disease or condition (i.e. so that the subject shows reduced signs or symptoms of the disease to be treated or prevented, or preferably no signs and symptoms of the disease).

The initial period of treatment may be up to 28 days e.g. up to 21 days or up to 16 days e.g. up to 14, 12, 10 days or 8 days. The initial period of treatment may also be greater than 2 days e.g. greater than 4, 6, 8, 10 or 12 days. For example, the initial treatment period may be e.g. 14 to 20 days e.g. 16 to 18 days or 11 to 14 days e.g. 12 or 13 days; or 8 to 10 days, for example 9 days or 8 days. Alternatively, the initial period of treatment may be in the range from 5 to 7 days, e.g. six days or seven days. Alternatively, the initial period of treatment may be shorter e.g. in the range from 2 to 4 days, such as 3 or 4 days. In an aspect, the initial period of treatment is one day less than a week or one day less than a fortnight. This enables the therapeutic dosage to be administered a week or two weeks later than the initial dose, which in cases where the initial dose and final therapeutic dose are administered in the presence of a physician enables the patient to attend a meeting with the physician on the same day.

In an aspect of the invention, the treatment regimen of the invention may be applied following an interruption in treatment or a treatment holiday during which treatment has been discontinued e.g. due to inadvertance or e.g. due to a need to reduce any immunosuppressant effect of the compounds in order to reduce the effect or duration of an opportunistic infection. The treatment holiday may be greater than e.g. 4 days, greater than 6, 8, 10, 12 or 14 days.

During the initial period of treatment, the first dose administered may be administered at a dosage between about 80 and 100-fold less than the standard daily dosage e.g. between about 60 and 80-fold less than the standard daily dosage e.g. between about 40 and 60-fold less than the standard daily dose, e.g. between about 20 and 40-fold less than the standard daily dosage e.g. between about 10 and 20-fold less than the standard daily dosage e.g. between about 2 and 10-fold less than the standard daily dosage. For example, the initial dosage may be administered at a value of about 3, 4, 5, 6 or 7 fold less than the standards daily dosage e.g. about 4 fold less.

The dosage is then increased, optionally stepwise towards the therapeutic dosage. For example the initial period may include up to 10 dosage increases, e.g. up to 8 dosage increases, e.g. up to 6 dosage increases, e.g. up to 5 dosage increases, up to 4 dosage increases or up to 3 dosage increases until the standard daily dosage is given. For example 1 to 10, e.g. 1 to 8, e.g. 2 to 8, e.g. 3 to 6 dosage increases may be given or 2 or 3 dosage increases. In an embodiment of the invention, only one dosage increase occurs before the standard daily dosage, e.g. the therapeutic dosage, is given.

The same dose may be given during the first 10-18 days of treatment before the dosage is increased e.g. the first 12 to 16 days of treatment. Following the first dosage increase, the same dose may be given during the next 10-18 days of treatment before the dosage is increased again or for a shorter period e.g. 2-6 days e.g. 4 days.

Alternatively, the same dose may be given during the first 1-5 days of treatment before the dosage is increased e.g. the first 2 to 4 days of treatment e.g. the first two or three days. Similarly, following the first dosage increase, the same dose may be given during the next 1-5 days of treatment e.g. the next 2 to 4 days of treatment e.g. the next two or three days before the dosage is increased again. This may also apply to the third and successive dosage increases until the standard daily dosage is reached. The dosage increase between steps may be constant or varying e.g. increasing.

Alternatively, the dosage may be increased stepwise daily in a defined incremental ratio up to the standard daily dosage of the S1 P receptor modulator or agonist. For example, each successive dose may be in the range 1.0-2.0 of the previous day's dose, for example in the range 1.2-1.8 or 1.4-1.6.

In an embodiment, the daily dosage is governed by a Fibonacci series i.e. the dosage given on a specific day is the sum of the dosages on the previous two days. In an aspect of this embodiment, some variation in this scheme is permitted. For example, the dosage on a given day may be the sum of the dosages on the two previous days ± 40%, for example ± 30%, for example ± 20% or ± 10%.

### Conditions to be treated

The dosage regimen of the invention may be used in conjunction with the use of the compounds of the invention in the treatment or prevention of any condition treatable by those compounds e.g. transplant rejection or an autoimmune condition.

Examples of conditions include transplant rejection, such as acute or chronic rejection of cell, tissue or organ allo- or xenografts or delayed graft function, graft versus host disease.

Further examples include e.g. Polymyositis, lupus nephritis, or psoriasis, rheumatoid arthritis, systemic lupus erythematosus, Subacute Cutaneous Lupus Erythematosus (SCLE), hashimoto's thyroidis, multiple sclerosis, myasthenia gravis, diabetes type I or II and the disorders associated therewith, vasculitis, pernicious anemia, Sjoegren syndrome, uveitis, psoriasis, Graves ophthalmopathy, alopecia areata and others, allergic diseases, e.g. allergic asthma, atopic dermatitis, allergic rhinitis/conjunctivitis, allergic contact dermatitis, inflammatory diseases optionally with underlying aberrant reactions, e.g. inflammatory bowel disease, Crohn's disease or ulcerative colitis, intrinsic asthma, inflammatory lung injury, inflammatory liver injury, inflammatory glomerular injury, atherosclerosis, osteoarthritis, irritant contact dermatitis and further eczematous dermatitises, seborrhoeic dermatitis, cutaneous manifestations of immunologically-mediated disorders, inflammatory eye disease, keratoconjunctivitis, myocarditis or hepatitis, ischemia/reperfusion injury, e.g. myocardial infarction, stroke, gut ischemia, renal failure or hemorrhage shock, traumatic shock, T cell lymphomas or T cell leukemias, infectious diseases, e.g. toxic shock (e.g. superantigen induced), septic shock, adult respiratory distress syndrome or viral infections, e.g. AIDS, viral hepatitis, chronic bacterial infection, or senile dementia. Examples of cell, tissue or solid organ transplants include e.g. pancreatic islets, stem cells, bone marrow, corneal tissue, neuronal tissue, heart, lung, combined heart-lung, kidney, liver, bowel, pancreas, trachea or oesophagus. For the above uses the required dosage will of course vary depending on the mode of administration, the particular condition to be treated and the effect desired.

Furthermore, the compounds are useful in cancer chemotherapy, particularly for cancer chemotherapy of solid tumors, e.g. breast cancer, or as an anti-angiogenic agent.

The dosage regimen of the present invention is particularly useful for treating patents at risk of cardiac side effects, for example patients at risk of heart failure, arrythmias, patients with high grade atrio-ventricular blocks or sick sinus syndrome, patients with a history of syncopal episodes, patients suffering from palpitations, or patients requiring or under beta blockers, or patients requiring or under anti-arrhythmic treatment, such as patients under treatment with Class Ia (e.g. quinidine, procainamide) or Class III anti-arrhythmic drugs (e.g., amiodarone, sotalol). The dosage regimen of the present invention may also be particularly useful for treating patents suffering from other, possibly related, conditions e.g dizziness and/or fatigue.

In these patients, the use of the dosage regime of the invention may ameliorate the risk of these cardiac side effects and other conditions e.g. to a level that is not clinically significant or to a level where the risk/benefit ratio is sufficiently low that treatment of the patient's condition using the compounds of the invention, with initial administration according to a dosage regime as described herein, would be judged to be beneficial to the patient by a physician of ordinary skill.

### Dosage

The standard daily dosage is selected to give the optimum balance of efficacy vs safety. According to the invention, the standard daily dosage e.g. the therapeutic dosage of the compound e.g. 2-amino-2-[4-(3-benzyloxyphenylthio)-2-chlorophenyl]ethyl-propane-1,3-diol (Compound A) is in the range from about 25 to about 0.1 mg.

In an embodiment, the standard daily dosage e.g. the therapeutic dosage may be in the range from about 0.8 - 3 mg per day e.g. about 1.0 - 2.0 mg per day e.g. 1, 1.2, 1.4, 1.5, 1.6, 1.8, 2.0, 2.2, 2.4 or 2.5 mg per day, e.g. about 2 mg per day.

These dosages may give an effect that is clinically meaningful in terms of reduction in absolute lymphocyte count, while avoiding or minimising possible adverse side effects e.g. cardiac safety (e.g. no or less pronounced heart rate reduction and/or AV blocks), renal safety (e.g. as measured by asymptotic elevation of liver enzymes) or pulmonary safety. In addition, the use of these dosages may also provide sufficient immunosuppression to treat the conditions affecting the patient but without substantially increasing the risk of opportunistic secondary infection.

For example in tests on healthy subjects, the maximum percent decrease (geometric mean, 95% confidence interval) from pre-dose blood lymphocyte levels during once daily dosing of compound A for 14 days (28 days for the 2 mg dose level) in healthy subjects was 39% for 0.3 mg, 65% for 0.6 mg, 74% for 1.2 mg, 83% for 2 mg and 77% for 3 mg. The corresponding reduction in the placebo group was 29%, which may be explained by the normal lymphocyte level variability and the definition of the endpoint. The blood lymphocyte levels typically returned to normal (above 1.0×10⁹/L) within two weeks after treatment discontinuation.

In the aspects relating to the kit, the kit may comprise just one low dose unit of medication at a dosage strength corresponding to an initial dosage of the S1P receptor modulator or agonist e.g. 0.1 mg, 0.2 mg, 0.3mg, 0.4mg, 0.5mg or 0.6mg. The kit may be formed to provide dosage units at an interval according to the patient's treatment schedule e.g. once weekly, once daily, twice daily, three times daily or four times daily.

In an aspect, the dosage forms in the kit are daily dosage forms. A patient may then take one unit of the low dose medication for a specified number of days and then, optionally, two or more units per day on subsequent days until therapy is commenced with a unit of medication that comprises the standard daily dose of the S1 P receptor agonist. This gives the advantage that only manufacture of a single additional dosage is required besides the therapeutic dosage.

In an alternative embodiment, the kit may comprise a number of low-dose units of medication with a range of dosage strengths so that the patient can be administered one dosage unit per day, but the amount of S1 P receptor modulator or agonist administered can be titrated upwards until therapy commences at the standard daily dosage. For example, the kit may comprise 2, 3 or 4 e.g. three different dosage forms. This gives the advantage that additional flexibility in the dosage regime is provided.

In an aspect, the kit may comprise a pack, e.g. a pack containing 1-5 e.g. 2-4 e.g. three different dosage forms. The pack may comprise individual storage portions each portion containing the patient's daily dosage for a given day during the course of treatment. The daily dosage may be made up of one or more of the different dosage forms. In an aspect of this embodiment, the kit comprises a blister pack containing 2-4 e.g. three different dosage forms in which the blisters in the pack contain the daily dosages for administration to the patient during the initial treatment phase, wherein the daily dosage is made up of one or more of the different dosage forms. In an aspect of this embodiment, the pack e.g. the blister pack may comprise a number of blisters corresponding to the number of days of the initial treatment period. In another aspect, the blister pack may also contain one or more blisters containing the final therapeutic dose e.g. so that the total treatment period including the low dosage and therapeutic dosage form lasts for a clinically convenient period of time e.g. one week or two weeks.

### Example 1

The effect on heart rate of 2-amino-2-[4-(3-benzyloxyphenylthio)-2-chlorophenyl]ethyl propane-1,3-diol (Compound A, or KRP203) was measured in healthy subjects over a period of 28 days and compared to placebo.

Multiple ascending doses of compound A were investigated in healthy subjects. 60 subjects in 5 cohorts (12 subjects per cohort) were dosed at 0.3 mg, 0.6 mg, 1.2 mg and 3 mg once daily for 14 days and at 2 mg for 28 days. In each cohort, subjects were randomised between compound A (9 subjects) and placebo (3 subjects).

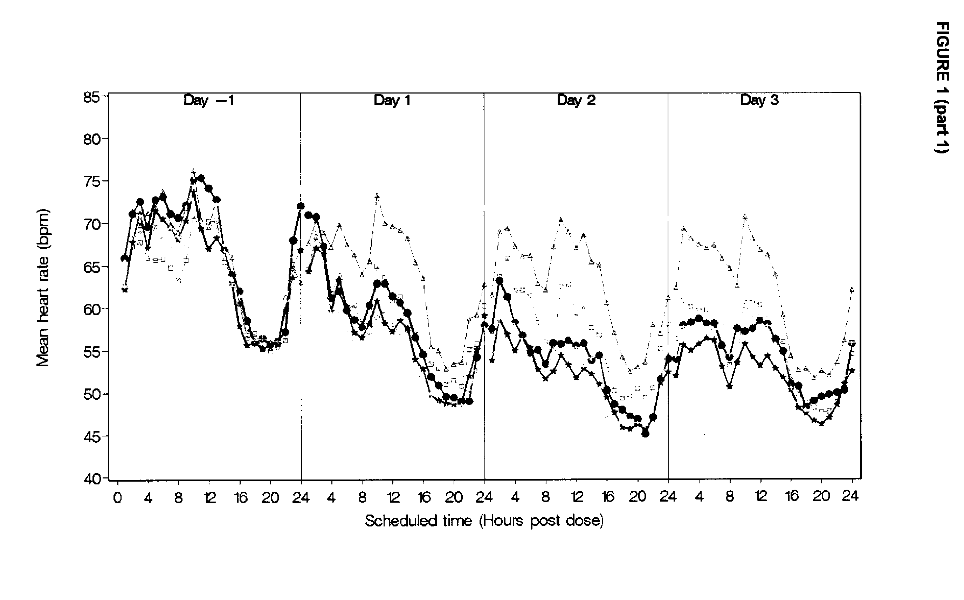

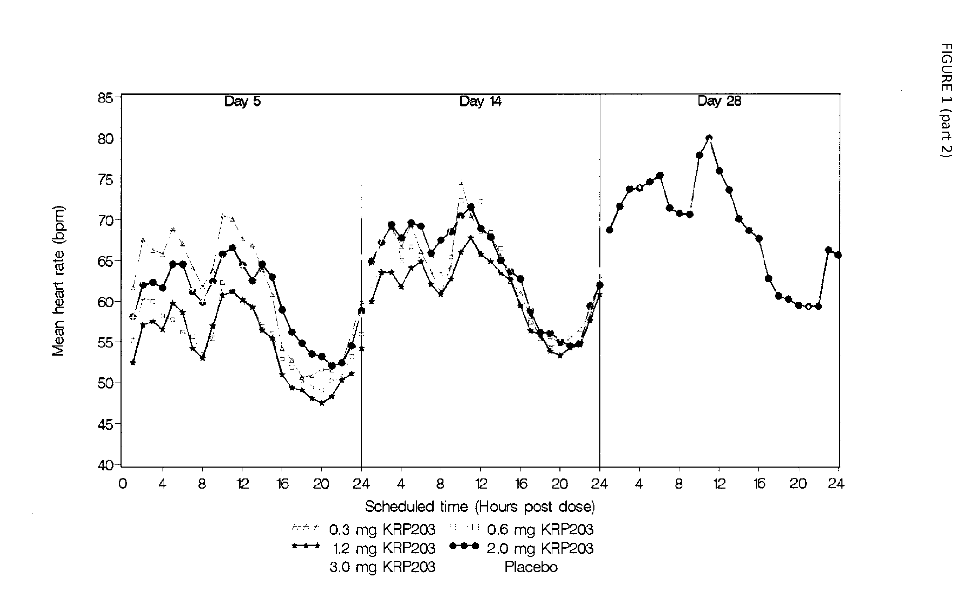

As seen in Figure 1 (part1 and part2), the decrease in heart rate following dosing was observed to increase with increasing dosage of compound A. This difference decreased as the trial progressed. Heart rates on Day 28 were similar for the 2 mg dose level and the placebo treated subjects.

Figure 1 (part1 and part2) shows the mean profile in hourly average heart rate by the indicated study day, starting by day (-1) where all received placebo, and continuing with day 1, day 2, day 3, day 5, and day 14. The day 28 graphic compares placebo and 2mg dose.

### Example 2

Utility of the dosage regimen in attenuating the negative chronotropic effect and/or providing other safety benefits e.g. reduction in number of heart pauses greater than 3 seconds may be demonstrated in standard animal or clinical tests, e.g. in accordance with the method described hereinafter.

A total of 56 healthy male volunteers were enrolled in a double blind, parallel, placebo controlled, dose titration, once daily, multiple oral dose study. 53 (95%) subjects completed the study.

Increasing doses of 2-amino-2-[4-(3-benzyloxyphenylthio)-2-chlorophenyl]ethyl-propane-1,3-diol (Compound A) starting at 0.3 mg o.d. or 0.5 mg o.d. and ending at the maximal therapeutic dose of 2 mg o.d. were administered to the subjects as specified in Table 1 below.

**Table 1**

| Study Period | Run-in | Treatment Period | | | | | | | | | | | | | | | | | | | | |
|---|---|---|---|---|---|---|---|---|---|---|---|---|---|---|---|---|---|---|---|---|---|---|
| Day | -1 | 1 | 2 | 3 | 4 | 5 | 6 | 7 | 8 | 9 | 10 | 11 | 12 | 13 | 14 | 15 | 16 | 17 | 18 | 19 | 20 | 21 |
| Titration group #1 (mg) | placebo | 0.3 | 0.3 | 0.3 | 0.3 | 0.6 | 0.6 | 0.6 | 0.6 | 0.9 | 0.9 | 0.9 | 0.9 | 12 | 12 | 12 | 12 | 2 | 2 | 2 | 2 | 2 |
| Titration group #2 (mg) | placebo | 0.5 | 0.5 | 0.5 | 0.5 | 0.5 | 0.5 | 0.5 | 0.5 | 0.5 | 0.5 | 0.5 | 0.5 | 0.5 | 0.5 | 12 | 1.2 | 1.2 | 12 | 2 | 2 | 2 |
| Fixed-dose group (mg) | placebo | 2 | 2 | 2 | 2 | 2 | 2 | 2 | 2 | 2 | 2 | 2 | 2 | 2 | 2 | 2 | 2 | 2 | 2 | 2 | 2 | 2 |
| Placebo | placebo | placebo, one daily | | | | | | | | | | | | | | | | | | | | |

Each subject participated in a maximal 21-day Screening period, a Baseline period (Day -2), Run-in period (Day -1), a 21-day treatment period, 14-day Follow up period (with a Follow up visit on Day 35) and study completion assessments on Day 42. Subjects were assigned to one of the four groups (#1 or #2 dose titration group, placebo- or fixed-dose group) and received the once daily (o.d.) treatment in a double blinded manner.

Subjects who met all the eligibility criteria at Screening entered the study center on the evening of Day -2 and remained in the center until 24 hours after the last dose (Day 22).

On Day -1 (Run-in), all subjects received placebo and underwent baseline assessments including 24 hour Holter monitoring.

All the study drugs were administered between 8:00 and 8:30, immediately after modest breakfast started 30 min prior to dosing. Dosing was performed as closely as practically possible between the subjects (e.g. within 15 min).

On Day 22, subjects were discharged from the study center after completing scheduled procedures and being evaluated by the Investigator. Subjects returned to the study center on Day 35 (two weeks after the last dose) for the Follow up visit and on Day 42 for End-of-study visit.

Subjects were assigned to one of the four treatment groups:
T#1 dose titration group (N=14)
T #2 dose titration group (N=14)
Plac: placebo (N=14)
Fix: fixed-dose group (N=14)

### Assessments and evaluations:

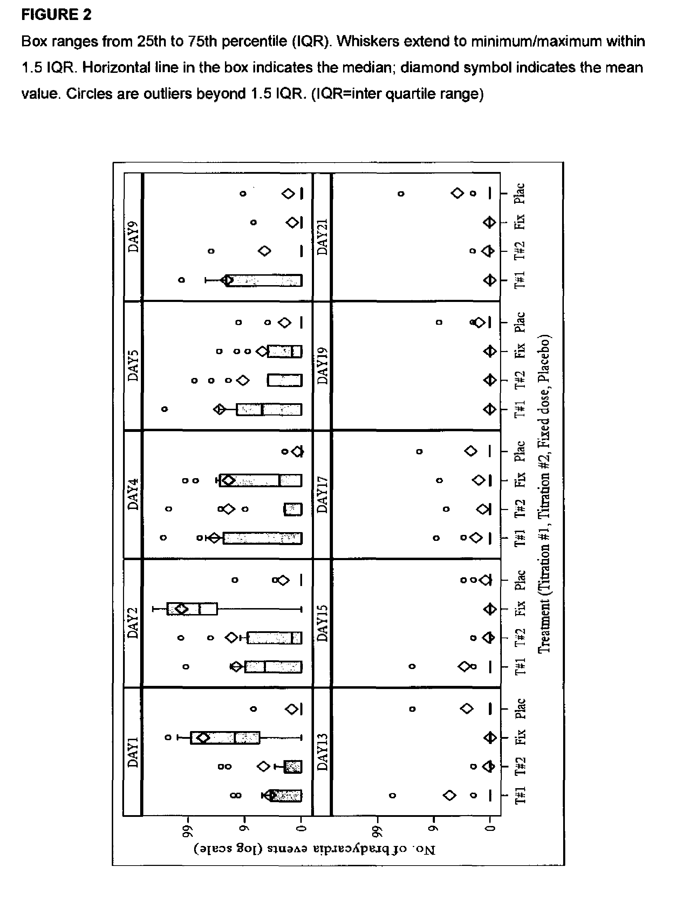

In Figure 2, the number of bradycardia episodes per day is shown for the 4 groups, i.e. T#1, T#2, Fix, and Plac, from DAY 1 up to DAY 21. Figure 2 shows the box-plots for the number of bradycardia episodes per day. Both titration regimens had less bradycardic episodes compared to fixed dose day 1. T #2 seemed more favorable compared to T#1 regarding magnitude and duration of effect observed with dose titration. However, T#1 and T#2 were not statistically different with respect to the number of bradycardia events in any of the days considered.

### Safety and tolerability

As expected, based on the mechanism of action for the active ingredient, i.e. Compound A, S1P agonist, the absolute lymphocyte count (ALC) decreased from baseline to Day 21 in all the active treatment groups. At the end of study visit the ALC levels had returned to near baseline values in the dose titration groups.

Both titration regimens were safe and well tolerated. No SAEs (severe adverse events) were reported. No discernable difference in the AE (adverse events) profile was observed between the Compound A dose titration groups and the placebo.

### Statistical methods

The primary variable, the daily number of bradycardia episodes as defined in the primary objective, were modeled by means of a repeated Poisson model adjusted for the number of bradycardia episodes at Baseline, the day, the treatment, and the treatment-by-day interaction. Generalized Estimating Equations were used to estimate the parameters of the model, including the mean (and 95% CI) number of bradycardia episodes for each treatment group on each day.

The primary objective of the study was assessed from the estimated ratio, obtained from the model above, between the mean number of bradycardia episodes on each 'dose increase day' in each titration regimen versus the mean number of bradycardia episodes on Day 1 in the fixed-dose group, and from the 95% CI for this ratio.

Other contrasts of interest were obtained from this model, e.g., to compare the titration regimens to placebo on each day.

The treatment effect in term of other measures of bradycardia, including the change from baseline in hourly (daily) minimum and mean heart rates, was also investigated using an appropriate model.

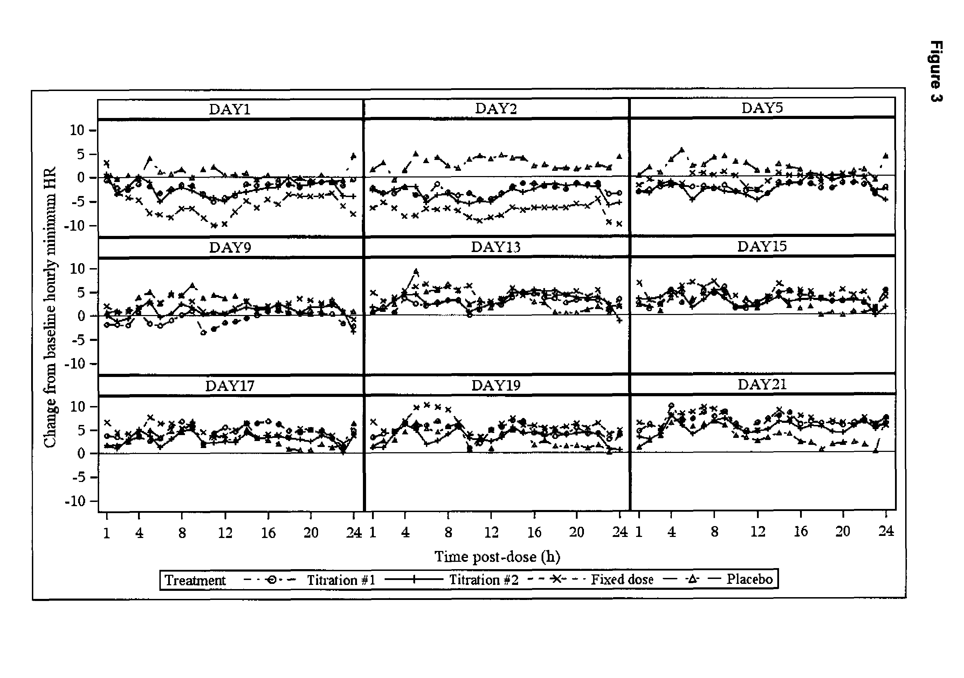

Figure 3 shows the hourly minimum HR (heart rate) change from baseline.

The fixed dose regimen had the maximum decrease in HR on Days 1 & 2 compared to the 2 titration regimens and resolved by day 5. After Day 13, all subjects showed no decrease in HR compared to matched baseline.

## Claims

1. A compound selected from: 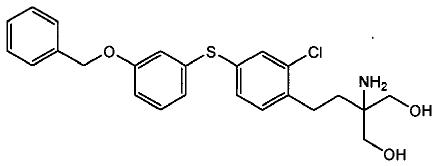 2-amino-2-[4-(3-benzyloxyphenylthio)-2-chlorophenyl]ethyl-propane-1,3-diol or a pharmaceutically acceptable salt, hydrate, or solvate, thereof,
and its corresponding phosphate derivatives: 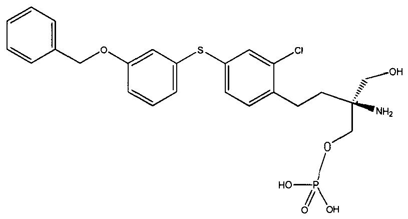 Phosphoric acid mono-{(S)-2-amino-4-[4-(3-benzyloxy-phenylsulfanyl)-2-chloro-phenyl]-2-hydroxymethyl-butyl}ester, or a pharmaceutically acceptable salt, hydrate, or solvate, thereof,
or 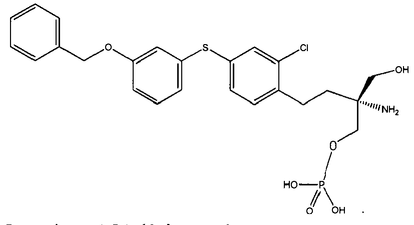 phosphoric acid mono-{(R)-2-amino-4-[4-(3-benzyloxy-phenylsulfanyl)-2-chloro-phenyl]-2-hydroxymethyl-butyl}ester
or a pharmaceutically acceptable salt, hydrate, or solvate thereof, for use in a method of therapeutic treatment or prevention of a condition treatable by the compound, optionally of an autoimmune condition, wherein said compound is administered at a dosage lower than the standard daily dosage of said compound during the initial period of treatment and then is increased, optionally stepwise, up to the standard daily dosage of said compound, and wherein said standard daily dosage is in the range from about 25 to about 0.1 mg.

2. The compound for use according to claim 1, wherein the compound is: 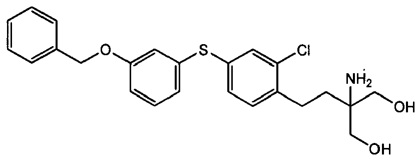 2-amino-2-[4-(3-benzyloxyphenylthio)-2-chlorophenyl]ethyl-propane-1,3-diol,
or a pharmaceutically acceptable salt, hydrate, or solvate thereof.

3. A compound for use according to any one of claims 1-2, or a pharmaceutically acceptable salt, hydrate, or solvate thereof, wherein said lower dosage is between 2 and 10-fold less than the standard daily dosage.

4. A compound for use, in accordance to any one of claims 1-2, or a pharmaceutically acceptable salt, hydrate, or solvate thereof, wherein said initial period is up to 28 days .

5. A kit containing units of medication of a compound or a pharmaceutically acceptable salt, hydrate, or solvate, thereof, as defined in any one of claims 1 to 2, of varying daily dosage, whereby said doses are lower than the standard daily dosage, wherein said standard daily dosage is in the range from about 25 to about 0.1 mg.

6. The kit of claim 5, wherein the units of medication are for a dosage regimen defined in any of the preceding claims.

## Patentansprüche

1. Verbindung, ausgewählt aus: 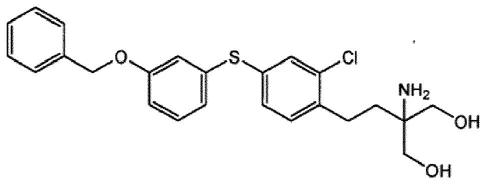 2-Amino-2-[4-(3-benzyloxyphenylthio)-2-chlorphenyl]ethylpropan-1,3-diol oder einem pharmazeutisch unbedenklichen Salz, Hydrat oder Solvat davon
und ihren dazugehörigen Phosphat-Derivaten: 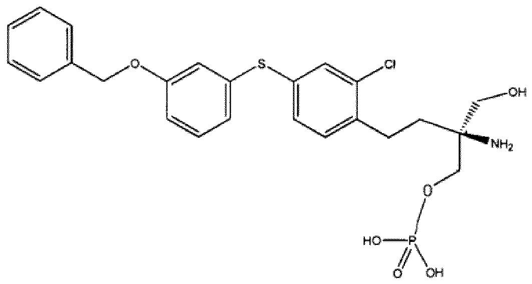 Phosphorsäure-mono-{(S)-2-amino-4-[4-(3-benzyloxyphenylsulfanyl)-2-chlorphenyl]-2-hydroxymethylbutyl}ester oder einem pharmazeutisch unbedenklichen Salz, Hydrat oder Solvat davon oder 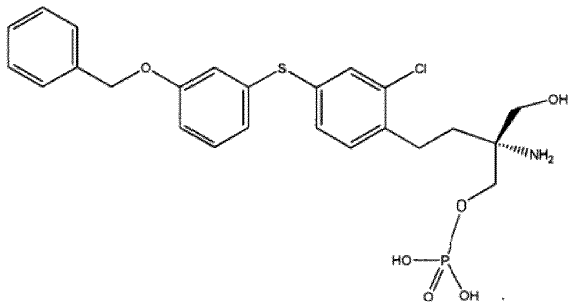 Phosphorsäure-mono-{(R)-2-amino-4-[4-(3-benzyloxyphenylsulfanyl)-2-chlorphenyl]-2-hydroxymethylbutyl} ester
oder einem pharmazeutisch unbedenklichen Salz, Hydrat oder Solvat davon zur Anwendung in einem Verfahren zur therapeutischen Behandlung oder Prävention einer mit der Verbindung behandelbaren Erkrankung, wahlweise einer Autoimmunerkrankung, wobei die Verbindung während der Anfangsphase der Behandlung in einer Dosierung verabreicht wird, die niedriger als die tägliche Standarddosierung der Verbindung ist und die danach, wahlweise schrittweise, bis auf die tägliche Standarddosierung der Verbindung erhöht wird, und wobei die tägliche Standarddosierung im Bereich von etwa 25 bis etwa 0,1 mg liegt.

2. Verbindung zur Anwendung nach Anspruch 1, wobei die Verbindung Folgendes ist: 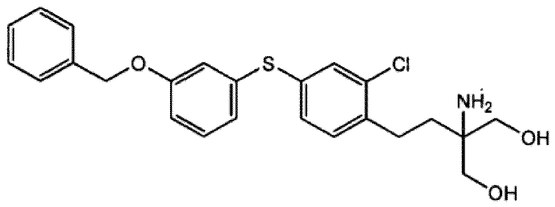 2-Amino-2-[4-(3-benzyloxyphenylthio)-2-chlorphenyl]ethylpropan-1,3-diol oder ein pharmazeutisch unbedenkliches Salz, Hydrat oder Solvat davon.

3. Verbindung zur Anwendung nach einem der Ansprüche 1-2 oder ein pharmazeutisch unbedenkliches Salz, Hydrat oder Solvat davon, wobei die niedrigere Dosierung zwischen 2 und 10 mal niedriger als die tägliche Standarddosierung ist.

4. Verbindung zur Anwendung nach einem der Ansprüche 1-2 oder ein pharmazeutisch unbedenkliches Salz, Hydrat oder Solvat davon, wobei diese Anfangsphase bis zu 28 Tage dauert.

5. Kit, das Medikamenteneinheiten einer Verbindung oder eines pharmazeutisch unbedenklichen Salzes, Hydrats oder Solvats davon, wie in einem der Ansprüche 1 bis 2 definiert, mit unterschiedlicher täglicher Dosierung enthält, wobei die Dosen niedriger sind als die tägliche Standarddosierung, wobei die tägliche Standarddosierung im Bereich von etwa 25 bis etwa 0,1 mg liegt.

6. Kit nach Anspruch 5, wobei die Medikamenteneinheiten für ein Dosierungsschema gedacht sind, das in einem der vorhergehenden Ansprüche definiert ist.

## Revendications

1. Composé choisi parmi : 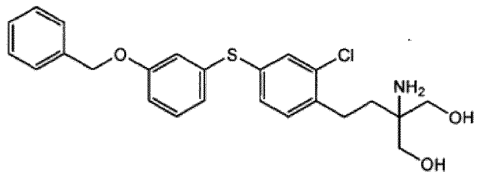 le 2-amino-2-[4-(3-benzyloxyphénylthio)-2-chlorophényl]éthyl-propane-1,3-diol ou un sel, hydrate ou solvate de celui-ci pharmaceutiquement acceptable, et ses dérivés phosphatés correspondants; 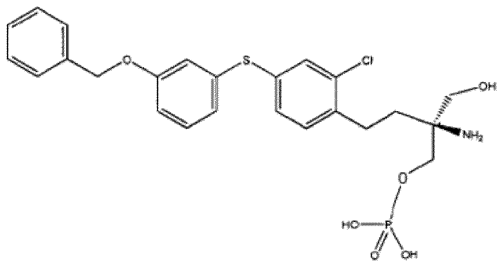 le mono-{(S)-2-amino-4-[4-(3-benzyloxy-phénylsulfanyl)-2-chloro-phényl]-2-hydroxyméthyl-butyl}ester de l'acide phosphorique, ou un sel, hydrate ou un solvate de celui-ci pharmaceutiquement acceptable, ou 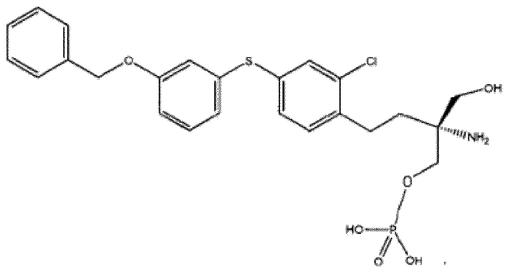 le mono-{(R)-2-amino-4-[4-(3-benzyloxy-phenylsulfanyl)-2-chloro-phényl]-2-hydroxyméthyl-butyl}ester de l'acide phosphorique ou un sel, hydrate ou solvate de celui-ci pharmaceutiquement acceptable, destiné à être utilisé dans une méthode de traitement thérapeutique ou de prévention d'un état traitable par le composé, éventuellement d'un état auto-immune, ledit composé étant administré à une dose qui est inférieure à la dose quotidienne normale dudit composé au cours de la période initiale du traitement, puis qui est augmentée, éventuellement par étapes, jusqu'à la dose quotidienne normale dudit composé, et ladite dose quotidienne normale étant dans la plage d'environ 25 à environ 0,1 mg.

2. Composé destiné à être utilisé conformément à la revendication 1, dans lequel le composé est : 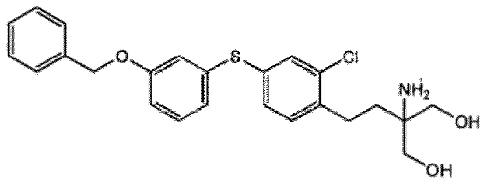 le 2-amino-2-[4-(3-benzyloxyphénylthio)-2-chlorophényl]éthyl-propane-1,3-diol, ou un sel, hydrate ou solvate de celui-ci pharmaceutiquement acceptable.

3. Composé destiné à être utilisé selon l'une quelconque des revendications 1 et 2, ou un sel, hydrate ou solvate de celui-ci pharmaceutiquement acceptable, ladite dose inférieure étant de 2 à 10 fois inférieure à la dose quotidienne normale.

4. Composé destiné à être utilisé conformément à l'une quelconque des revendications 1 et 2, ou un sel, hydrate ou solvate de celui-ci pharmaceutiquement acceptable, ladite première période pouvant aller jusqu'à 28 jours.

5. Kit contenant des unités de médication d'un composé ou d'un sel, hydrate ou solvate de celui-ci pharmaceutiquement acceptable, tel que défini dans l'une quelconque des revendications 1 et 2, à dose quotidienne variable de sorte que lesdites doses sont inférieures à la dose quotidienne normale, ladite dose quotidienne normale étant dans la plage d'environ 25 à environ 0,1 mg.

6. Kit selon la revendication 5, dans lequel les unités de médication sont associées à un régime de doses défini dans l'une quelconque des revendications précédentes.
